# EUROPÄISCHE PATENTSCHRIFT

(11) **EP 2 054 118 B1**
(45) Veröffentlichungstag und Bekanntmachung des Hinweises auf die Patenterteilung: **21.07.2010**
(21) Anmeldenummer: 07786605.1
(22) Anmeldetag: 07.08.2007
(51) Int. Cl.: A61M 25/10, A61M 29/02

(54) **VORRICHTUNG ZUR VERRINGERUNG ODER ENTFERNUNG VON STENOSEN**
DEVICE FOR REDUCING OR REMOVING STENOSES
DISPOSITIF POUR DIMINUER OU ÉLIMINER LES STÉNOSES

(30) Priorität: 21.08.2006 DE 102006039236
(43) Veröffentlichungstag der Anmeldung: 06.05.2009
(73) Patentinhaber: Variomed AG, 9496 Balzers (LI)
(72) Erfinder: STENGEL, Max, 76646 Bruchsal (DE)
(74) Vertreter: Manitz, Finsterwald & Partner GbR
(86) Internationale Anmeldenummer: PCT/EP2007/006980
(87) Internationale Veröffentlichungsnummer: WO 2008/002270

(56) Entgegenhaltungen:
- WO-A-98/47558
- WO-A-98/57592

## Beschreibung

Die vorliegende Erfindung betrifft eine Vorrichtung zum Verringern oder Entfernen einer in einem Blutgefäß vorhandenen Stenose mit einem Arbeitskatheter mit einem Lumen, das einen sich vom distalen Ende bis zum proximalen Ende des Arbeitskatheters erstreckenden ersten Arbeitskanal zum Einführen eines Instruments zum Verringern oder Entfernen der Stenose bildet, wobei ein von dem Arbeitskatheter getrennt ausgebildeter Ballonkatheter vorgesehen ist, der im Bereich seines distalen Endes einen Abdichtungsballon umfasst, wobei der Ballonkatheter zwei voneinander getrennt ausgebildete Lumen umfasst, von denen eines zur Dilatation des Abdichtungsballons mit diesem verbunden ist, während das andere einen sich vom distalen Ende bis zum proximalen Ende des. Ballonkatheters erstreckenden zweiten Arbeitskanal bildet. Weiterhin wird ein Verfahren zum Verringern oder Entfernen einer in einem Blutgefäß vorhandenen Stenose exemplarisch beschrieben, bei dem ein Arbeitskatheter über einen Zugang in das Blutgefäß eingeführt wird, bis das distale Ende des Arbeitskatheters im Bereich proximal der Stenose zu liegen kommt, wobei der Arbeitskatheter ein Lumen aufweist, das einen sich vom distalen Ende bis zum proximalen Ende des Arbeitskatheters erstreckenden ersten Arbeitskanal bildet.

Vorrichtungen und Verfahren dieser Art werden beispielsweise zur Entfernung von Stenosen in der Arteria carotis und insbesondere in der Arteria carotis interna verwendet. Dabei kann das Aufweiten der Stenose beispielsweise durch eine Ballondilatation erfolgen, wobei zusätzlich ein Stent im Bereich der Stenose abgesetzt werden kann, um den aufgeweiteten Zustand dauerhaft sicherzustellen. Weiterhin ist es auch möglich, zumindest Teile der Ablagerungen abzutragen und abzusaugen.

Die WO 98/47558 A offenbart einen Kardioplegiekatheter zum Einführen in die linke Herzkammer und von dieser in die aufsteigende Aorta, wobei am distalen Ende des Katheters ein Ballon zum Verschließen der Aorta vorgesehen ist. Ferner befindet sich am distalen Ende die Öffnung eines Arbeitskanal. Das Kardioplegiekathetersystem kann zwei getrennte, parallel verlaufende Katheter umfassen, wobei das distale Ende des zweiten Katheters über das distale Ende des ersten Katheters hinausreicht und daher seitlich am Ballon vorbeiführt.

Grundsätzlich ist es problematisch, dass sowohl bei der Ballondilatation, als auch beim Platzieren des Stents oder beim mechanischen Bearbeiten der Stenose Ablagerungen gelöst werden können, die sich aufgrund der antegraden Strömung in der Aorta carotis interna in Richtung zum Gehirns bewegen und dort einen Schlaganfall auslösen können. Dies muss daher unter allen Umständen vermieden werden.

Bekannte Vorrichtungen und Verfahren verwenden daher oftmals ein so genanntes Protektionssystem, durch das distal der Stenose die Arteria carotis interna blockiert wird, so dass sich bei der Behandlung der Stenose ablösende Teilchen nicht in Richtung des Gehirns transportiert werden können. Problematisch an diesen Vorrichtungen ist jedoch, dass vor Wirksamwerden des Protektionssystems dieses zunächst durch die Stenose hindurch geführt werden muss, um in dem Bereich distal der Stenose wirksam werden zu können. Beim Hindurchführen durch die Stenose besteht jedoch die Gefahr, dass bereits Teile der Stenose abgelöst werden und in Richtung des Gehirns wandlern.

Es sind daher bereits Vorrichtungen bekannt, bei denen über den Arbeitskanal des Arbeitskatheters ein Führungsdraht geführt wird, an dessen distalem Ende ein Protektionselement beispielsweise in Form eines Ballons vorgesehen ist. Dieses Protektionselement wird über den Arbeitskanal des Katheters in die Arteria carotis externa eingeführt, um dort den Blutfluss zu stoppen und, nach Verbinden des proximalen Endes des Arbeitskanals mit einer Vene, in der Arteria carotis interna eine Flussumkehr zu erreichen. Dazu besitzt der Arbeitskatheter an seinem distalen Ende einen Ballon, mit dem die Arteria carotis communis blockiert wird, so dass ein Rückstrom erzeugt wird, der von der Arteria carotis interna durch den Arbeitskanal des Arbeitskatheters hindurch erfolgt.

Problematisch an dieser Vorrichtung ist, dass der üblicherweise im Bereich des Oberschenkels oder der Leiste angelegte Punktionskanal zum Einbringen des Arbeitskatheters relativ groß ausgebildet sein muss, da der Arbeitskatheter einen relativ großen Durchmesser besitzt. Dies liegt daran, dass der Arbeitskatheter sowohl das Blockierelement zum Blockieren der Arteria carotis communis als auch einen Arbeitskanal umfassen muss, durch den sowohl das Blockierelement zum Blockieren der Arteria carotis externa als auch das Instrument zum Entfernen der Stenose geführt werden muss, wobei gleichzeitig ein ausreichender retrograder Blutfluss durch den Arbeitskanal gewährleistet sein muss. Weiterhin ist an der Außenseite des Arbeitskatheters üblicherweise noch eine Außenhülle (Schleuse) vorgesehen, durch die der außen liegende Ballon beim Einführen des Arbeitskatheters abgedeckt wird. Durch diese Schleuse wird der Durchmesser des Arbeitskatheters weiter vergrößert.

Problematisch an diesen großen Punktionsstellen ist, dass deren Abheilung oftmals nur relativ schlecht erfolgt und dadurch auch das Entzündungsrisiko vergrößert wird. Weiterhin besteht bei der Verwendung eines einzigen Arbeitskanals das Problem, dass durch die durch den Arbeitskanal geführten Instrumente der Arbeitskanal oftmals fast vollständig belegt ist, so dass nicht in allen Fallen ein ausreichender retrograder Blutfluss durch den Arbeitskanal gewährleistet ist.

Es ist eine Aufgabe der vorliegenden Erfindung, eine Vorrichtung der eingangs genannten Art so auszubilden, dass die genannten Nachteile vermieden werden.

Ausgehend von einer Vorrichtung der eingangs genannten Art wird diese Aufgabe dadurch gelöst, dass der Abdichtungsballon zum seitlichen Umgreifen des distalen Endbereichs des Arbeitskatheters mit einem unsymmetrischen Querschnitt ausgebildet ist, so dass bei nebeneinander in das Blutgefäß eingebrachtem Arbeitskatheter und Ballonkatheter durch den dilatierten Abdichtungsballon eine Abdichtung zwischen den Außenseite des Arbeitskatheters und des Ballonkatheters sowie der Innenwand des Blutgefäßes gebildet wird.

Exemplarisch wird ein Verfahren beschrieben, bei dem ein von dem Arbeitskatheter getrennt ausgebildeter Ballonkatheter über einen zweiten, von dem ersten Zugang getrennten Zugang in das Blutgefäß eingeführt wird, bis das distale Ende des Ballonkatheters im Bereich neben dem distalen Ende des Arbeitskatheters zu liegen kommt, wobei der Ballonkatheter im Bereich seines distalen Endes einen Abdichtungsballon mit einem unsymmetrischen Querschnitt sowie zwei voneinander getrennt ausgebildet Lumen umfasst, von denen eines zur Dilatation des Abdichtungsballons mit diesem verbunden ist, während das andere einen sich vom distalen Ende bis zum proximalen Ende des Ballonkätheters erstreckenden zweiten Arbeitskanal bildet, der Abdichtungsballon über das Lumen so dilatiert wird, dass der distale Endbereich des Arbeitskatheters von dem Abdichtungsballon so umgriffen wird, dass eine Abdichtung zwischen den Außenseiten des Arbeitskatheters und des Ballonkatheters sowie der Innenwand des Blutgefäßes gebildet wird, und wobei über den ersten Arbeitskanal ein Instrument zum Verringern oder Entfernen der Stenose in das Blutgefäß eingeführt wird.

Es erfolgt somit eine Aufteilung in zwei voneinander getrennte Katheter, nämlich einen Arbeitskatheter und einen Ballonkatheter, die über zwei getrennte Zugänge in das Blutgefäß eingeführt werden. Durch die Aufteilung in zwei getrennte Katheter können die beiden erforderlichen Zugänge jeweils deutlich kleiner ausgebildet werden als es bei einem gemeinsamen Katheter der Fall ist. Probleme bei der Heilung der entsprechenden Punktionsstellen werden dadurch zuverlässig vermieden. Grundsätzlich ist es dabei möglich, jeweils einen arteriellen Zugang pro Bein zu legen oder beide Zugänge ipsilateral, jedoch getrennt voreinander, vorzugehen.

Weiterhin wird durch die Aufteilung in zwei Arbeitskanäle erreicht, dass durch den ersten Arbeitskanal ein ständiger retrograder Blutfluss aufrechterhalten werden kann, durch den gewährleistet ist, dass sich bei der Dilatation der Stenose ablösende Teilchen nicht zum Gehirn wandern, sondern zuverlässig abgesaugt werden, während gleichzeitig durch den zweiten Ärbeitskanal ein schwächerer antegrader Blutfluss aufrechterhalten werden kann. Durch diesen zweiten Arbeitskanal ist daher die Gabe von Kontrastmitteln, Medikamenten oder NaCl ohne Risiko der Embolieverschleppung möglich.

Da an dem Arbeitskatheter kein außen liegender Ballon vorgesehen ist ist am Arbeitskatheter keine außen liegende Schleuse erforderlich, so dass demgemäß der erste Arbeitskanal mit einem vergrößerten Lumen ausgebildet sein kann, was die Handhabbarkeit beispielsweise bei der Ballondilatation sowie beim Einsetzen eines Stents erleichtert und gleichzeitig gewährleistet, dass ein ständiger Rückstrom durch den ersten Arbeitskanal aufrechterhalten bleibt. Selbst wenn der erste Arbeitskanal beim Einführen eines Stents oder eines Ballons kurzzeitig blockiert sein sollte, wird durch den zweiten Arbeitskanal ein Druckaufbau in der Arteria carotis interna vermieden. Ein entsprechender Druckaufbau wird auch in der Arteria carotis externa vermieden, da eine Blockierung der Arteria carotis externa überflüssig ist.

Durch die spezielle Ausbildung des Abdichtungsballons zum seitlichen Umgreifen des distalen Endbereichs des Arbeitskatheters ist sichergestellt, dass der durch den ersten Arbeitskanal aufgebaute Rückstrom zuverlässig aufrechterhalten bleibt, so dass während der Behandlung der Stenose eventuell abgelöste Teilchen zusammen mit dem Rückstrom durch den ersten Arbeitskanal abgeführt werden. Durch die Verwendung eines unsymmetrischen Querschnitts kann das abdichtende Umgreifen des Arbeitskatheters durch den Abdichtungsballon verbessert werden. Durch die spezielle Ausbildung des Abdichtungsballons ist nur ein einziger Ballon zum Abdichten der Arteria carotis interna erforderlich, obwohl zwei getrennte Kathetersysteme verwendet werden. Die Arteria carotis externa ist auf diese Weise ebenfalls von der Stenose abgetrennt, ohne dass jedoch eine Blockierung der Arteria carotis externa erforderlich wäre.

Nach einer bevorzugten Ausführungsform der Erfindung umfasst der Abdichtungsballon eine Vielzahl von Einzelballons. Durch diese Aufteilung des Abdichtungsballons kann das Umgreifen des Arbeitskatheters verbessert und eine automatische Ausrichtung des Ballonkatheters zu dem Arbeitskatheter erreicht werden.

Vorteilhaft ist eine venöse Schleuse vorgesehen, über die der erste Arbeitskanal mit einer Vene verbindbar ist. Durch die unterschiedlichen Druckverhältnisse in Vene und Arterie wird erreicht, dass durch den ersten Arbeitskanal ein ständiger Rückstrom aufrechterhalten wird, der abgelöste Teilchen der Stenose zuverlässig abtransportiert. Grundsätzlich ist es jedoch auch möglich, dass der Rückstrom durch den Arbeitskanal nicht in eine Vene sondern beispielsweise nach außen abgeführt wird.

Nach einer weiteren bevorzugten Ausführungsform der Erfindung sind der erste und/oder der zweite Arbeitskanal zum Aufnehmen eines Führungdrahtes ausgebildet. Sowohl der Arbeitskatheter als auch der Ballonkatheter können somit über einen zunächst gelegten Führungsdraht an die gewünschte Stelle proximal der Stenose eingeführt werden. Nach erfolgter Platzierung wird der Führungsdraht in üblicher Weise entfernt, so dass der erste und/ oder der zweite Arbeitskanal für die weitere Verwendung zur Verfügung stehen.

Bevorzugt ist der zweite Arbeitskanal zum Einspritzen von Flüssigkeit, insbesondere von Kontrastmitteln, von Medikamenten oder von NaCl ausgebildet. Hierzu ist beispielsweise ein seitlicher Anschluss an den zweiten Arbeitskanal im Bereich dessen proximalen Endes vorgesehen. Obwohl durch den ersten Arbeitskanal ein ständiger Rückstrom in retrograder Richtung vorhanden ist, der ein zuverlässiger Abführen eventuell abgelöster Teilchen der Stenose gewährleistet, kann gleichzeitig durch den zweiten Arbeitskanal ein antegrader Strom vorhanden sein, über den eine Einspritzung in Richtung zur Stenose hin möglich ist.

Nach einer weiteren bevorzugten Ausführungsform ist eine Druckerzeugungseinheit zum Erzeugen eines konstanten Drucks vorgesehen, die über das mit dem Abdichtungsballon verbundene Lumen des Ballonkatheters an dem Abdichtungsballon angeschlossen ist, und durch die nach Dilatation des Abdichtungsballons der Druck innerhalb des Abdichtungsballons im Wesentlichen konstant gehalten wird. Durch die Druckerzeugungseinheit wird sichergestellt, dass auch bei einem Abfallen des Drucks im Abdichtungsballon, beispielsweise durch eine Leckage, die Abdichtung zwischen den Kathetern und der Gehäuseinnenwand sichergestellt ist.

Bevorzugt kann die Druckerzeugungseinheit beispielsweise einen Druckausgleichsbehälter umfassen, dessen Lumen insbesondere wesentlich größer ist als das Lumen des Abdichtungsballons. Da der Abdichtungsbal-Ion üblicherweise ein sehr geringes Volumen besitzt, kann durch einen entsprechend großen Ausgleichsbehälter in einfacher Weise ein nahezu konstanter Druck im Abdichtungsballon eingehalten werden. Geringe Druckschwankungen im Abdichtungsballon können durch das große Volumen des Druckausgleichsbehälters leicht kompensiert werden.

Nach einer weiteren vorteilhaften Ausführungsform der Erfindung besitzt der erste Arbeitskanal einen größeren Durchmesser als der zweite Arbeitskanal. Da durch den ersten Arbeitskanal sowohl das Instrument zum Entfernen der Stenose geführt wird als auch gleichzeitig ein ausreichender Querschnitt für den Rückstrom zur Verfügung gestellt werden soll, durch den zweiten Arbeitskanal hingegen lediglich die optionale Zufuhr von Kontrastmittel oder dergleichen vorgenommen wird, ist es vorteilhafte, wenn der erste Arbeitskanal einen größeren Durchmesser als der zweite Arbeitskanal besitzt. Darüber hinaus ist dadurch sichergestellt, dass in der Arteria carotis interna die gewünschte Flussumkehr in allen Fällen auf rechterhalten bleibt.

Nach einer weiteren bevorzugten Ausführungsform der Erfindung besitzt der Arbeitskatheter einen Durchmesser von ca. 5 bis 9 French, insbesondere von ca. 6 bis 8 French, bevorzugt von ca. 7 French. Der Ballonkatheter kann bevorzugt einen Durchmesser von 10 bis 14 mm, insbesondere von ca. 12 mm besitzen. Insbesondere für den Arbeitskatheter kann somit der Durchmesser des Punktionskanals minimiert werden, wodurch die Heilungsbedingungen für die Punktionsstelle deutlich verbessert werden.

Bevorzugt besitzt der Abdichtungsballon eine axiale Länge von ca. 0,5 bis 3 cm, insbesondere von ca. 1 bis 1,5 cm. Diese Längen des Abdichtungsballons sind üblicherweise ausreichend, um einen optimalen Kompromiss zwischen Abdichtung und Platzbedarf zu gewährleisten.

Nach einer weiteren bevorzugten Ausführungsform der Erfindung besteht der Abdichtungsballon aus elastischem Material. Grundsätzlich ist es auch möglich, dass der Abdichtungsballon aus unelastischem Material oder aus halbelastischem Material besteht. Wesentlich ist, dass der Abdichtungsballon von seinem Material und seinen sonstigen Eigenschaften her, beispielsweise seiner Materialdicke, so gewählt ist, dass ein möglichst weitgehendes Umschließen des Arbeitskatheters bei der Ballondilatation erfolgt und dass das Ballonmaterial insbesondere auch alle zwischen dem Arbeitskatheter und der Gefäßinnenwand vorhandenen Ritzen ausfüllt, so dass die Abdichtung möglichst vollständig ist.

Bevorzugt kann dazu beispielsweise der maximale Durchmesser des Abdichtungsballons größer als der Durchmesser des Blutgefäßes sein. Dies führt dazu, dass bei noch nicht vollständig dilatiertem Ballon die Ballonhaut nicht unter Spannung steht und sich somit leichter an die Außenkontur des Arbeitskatheters sowie die Innenseite des Gefäßes anschmiegen kann und insbesondere auch in schmale Ritzen zwischen Arbeitskatheter und Gehäuseinnenwand schlupfen kann.

Weitere vorteilhafte Ausführungsformen sind in den Unteransprüchen angegeben.

Die Erfindung wird nachfolgend anhand von Ausführungsbeispielen unter Bezugnahme auf die Zeichnungen näher beschrieben; in diesen zeigen:
- Fig. 1: eine stark vereinfachte Darstellung eines Arbeitskatheters, der durch die Arteria femoralis in die Arteria carotis interna eingeführt ist,
- Fig. 2: die Darstellung nach Fig. 1 mit zusätzlich eingeführtem Ballonkatheter,
- Fig. 3: eine erfindungsgemäß ausgebildete Vorrichtung mit Ar- beitskatheter und Ballonkatheter in vollständig eingebrach- tem Zustand,
- Fig. 4: eine Detailansicht einer erfindungsgemäß ausgebildeten Vorrichtung,
- Fig. 5: einen schematischen Querschnitt durch die Arteria carotis interna mit einer erfindungsgemäß ausgebildeten Vorrich- tung gemäß einer ersten Ausführungsform und
- Fig. 6: eine Ansicht nach Fig. 5 mit einer erfindungsgemäß ausge- bildeten Vorrichtung gemäß einer zweiten Ausführungs- form.

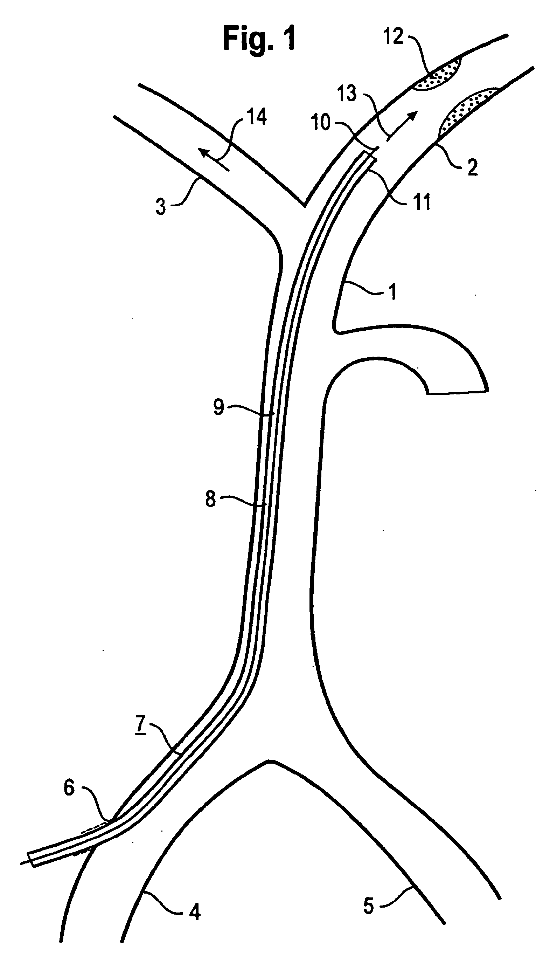

Fig. 1 zeigt in stark vereinfachter Darstellung die Arteria carotis communis 1, welche sich zum einen in die Arteria carotis interna 2 sowie die Arteria carotis externa 3 verzweigt und zum anderen mit den beiden Zweigen 4, 5 der Arteria femoralis in Verbindung steht.

In den Zweig 4 der Arteria femoralis ist über einen schematisch dargestellten Punktionskanal ein Zugang 6 für einen Arbeitskatheter 7 geschaffen, der vom Zugang 6 bis zu der Arteria carotis interna 2 erstreckt. Der Arbeitskatheter 7 umfasst ein Lumen 8, das einen ersten Arbeitskanal 9 des Arbeitskatheters 7 bildet. In dem ersten Arbeitskanal 9 ist ein Führungsdraht 10 angeordnet, über den der Arbeitskatheter 7 in seine in Fig. 1 dargestellte Position eingeführt wurde.

Distal zum distalen Ende 11 des Arbeitskatheters 7 ist in der Arteria carotis interna 2 eine Stenose 12 vorhanden. Weiterhin ist durch Pfeile 13, 14 der üblicherweise vorhandene antegrade Strömung des Bluts in der Arteria carotis interna 2 sowie der Arteria carotis externa 3 angedeutet.

Die Verwendung einer erfindungsgemäßen Vorrichtung wird unter Bezugnahme auf die Fig. 1 sowie die weiteren Figuren nun näher beschrieben.

Der Arbeitskatheter 7 wird zunächst über den gelegten Führungsdraht 10 ausgehend von dem Zugang 6 über den Zweig 4 der Arteria femoralis in üblicher Weise eingeführt, bis sein distales Ende 11 proximal von der Stenose 12 zu liegen kommt, wie es in Fig. 1 dargestellt ist. Da es sich bei dem Arbeitskatheter 7 lediglich um einen einfachen Katheter mit einem innen liegenden ersten Arbeitskanal 9 handelt, kann für den Zugang 6 beispielsweise eine Gefäßschleuse 6 bis 7 French oder ein Führungskatheter 7 bis 8 French verwendet werden.

Nach erfolgter Positionierung kann der Führungsdraht 10 entfernt werden. Die Stenose 12 ist von dem Einführen des Arbeitskatheters 11 nicht betroffen, so dass kein Risiko für eine Embolie besteht.

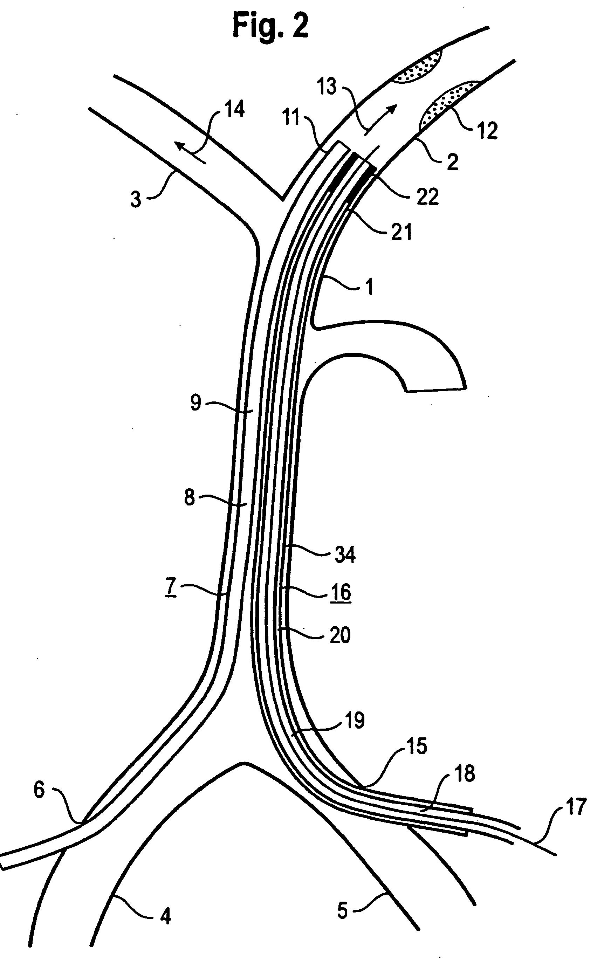

Anschließend wird über einen zweiten Zugang 15 (Fig. 2) in dem Zweig 5 der Arteria femoralis ein Ballonkatheter 16 eingeführt und parallel zu dem Arbeitskatheter 7 bis in die Arteria carotis interna 2 vorgeschoben. Das Einführen des Ballonkatheters 16 erfolgt dabei wiederum in üblicher Weise über einen Führungsdraht 17. Da zuvor bereits der Arbeitskatheter 7 gelegt wurde, ist diese zweite Sondierung problemlos möglich.

Der Ballonkatheter 16 umfasst ein innen liegendes Lumen 18, durch das ein zweiter Arbeitskanal 19 gebildet wird und in dem der Führungsdraht 17 beim Einführen des Ballonkatheters 16 angeordnet ist.

Weiterhin umfasst der Ballonkatheter 16 ein außen liegendes Lumen 20, das mit einem am distalen Ende 21 des Ballonkatheters 16 angeordneten Abdichtungsballon 22 in Verbindung steht. Zur Bildung des Lumens 20 ist an der Außenseite des Ballonkatheters 16 eine Außenhülle (Schleuse) 34 vorgesehen, die mit dem Abdichtungsballon 22 verbunden ist.

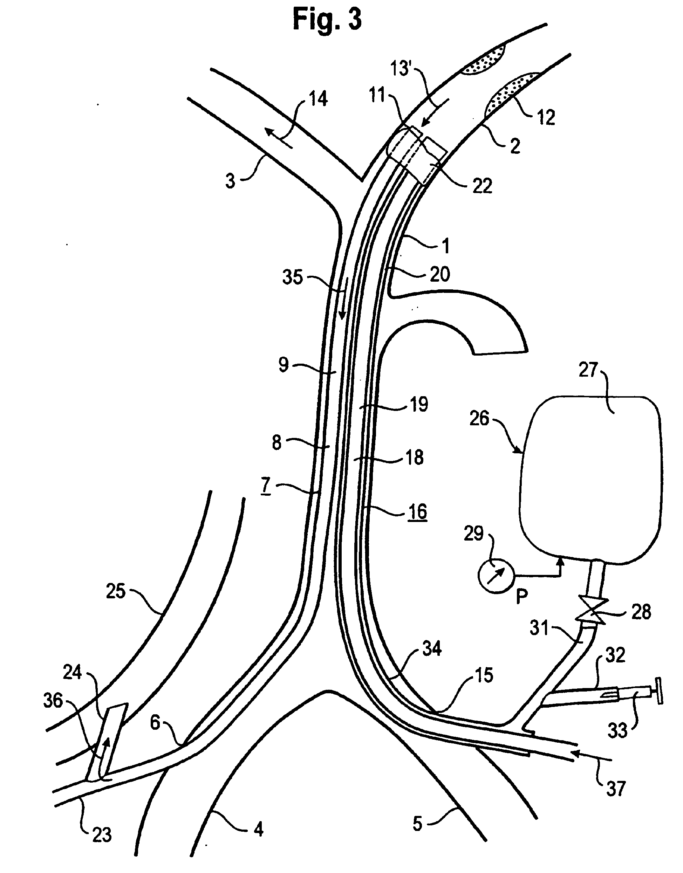

Wie in Fig. 3 angedeutet ist, wird das proximale Ende 23 des ersten Arbeitskanals 9 über einen Shunt 24 mit einer Vene 25, beispielsweise der Vena femoralis verbunden.

Weiterhin ist in Fig. 3 eine Druckerzeugungseinheit 26 dargestellt, die einen Druckausgleichsbehälter 27 umfasst, der über ein Ventil 28 mit dem Lumen 20 des Ballonkatheters 16 verbunden ist. An dem Druckausgleichsbehälter 27 ist darüber hinaus eine Anzeigeeinheit 29 (Manometer) zur Anzeige des in dem Druckausgleichsbehälter 27 vorhandenen Drucks angeschlossen. Der Druckausgleichsbehälter 27 ist beispielsweise mit NaCl gefüllt. Weiterhin kann ein Druckerzeuger an dem Druckausgleichsbehälter 27 angeschlossen sein, über den ein gewünschter Druck erzeugt und konstant gehalten werden kann. Der gewünschte Druck kann aber auch durch einmaliges Befüllen des Druckausgleichsbehälters 27 erzeugt werden.

Die Druckerzeugungseinheit 26 ist mit dem Lumen 20 des Ballonkatheters 16 über eine Y-Verbindung 30 verbunden, an deren einem Ast 31 die Druckerzeugungseinheit 26 und am anderen Ast 32 ein Anschluss für eine Spritze 33 vorgesehen ist. Über die Spritze 33 kann beispielsweise NaCl über das Lumen 20 zu dem Abdichtungsballon 22 des Ballonkatheters 16 zugeführt werden, so dass sich dieser ausdehnt und seine in Fig. 3 dargestellte vollständig dilatierte Form annimmt. Nach der vollständigen Dilatation des Abdichtungsballons 22 wird der Druck in diesem durch die Druckerzeugungseinheit 26 aufrechterhalten.

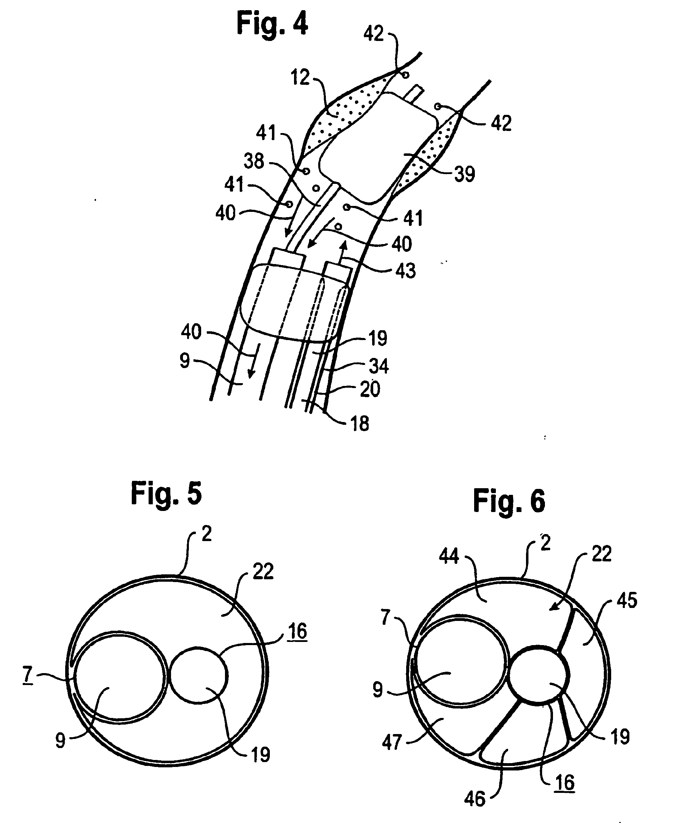

Durch die Dilatation des Abdichtungsballons 22 dehnt sich dieser so aus, dass er das distale Ende 11 des Arbeitskatheters 7 umgreift, wie es insbesondere aus den Fig. 5 und 6 zu erkennen ist. Der Abdichtungsballon 22 dehnt sich dabei so aus, dass er zum einen an der Innenwand der Arteria carotis interna 2 sowie an der Außenseite des Arbeitskatheters 7 anliegt und zum anderen auch in die Engstellen zwischen der Innenwand der Arteria carotis interna 2 und dem Arbeitskatheter 7 weitgehend vollständig eindringt. Auf diese Weise wird eine vollständige Blockierung der Arteria carotis interna 2 durch den Abdichtungsballon 22 erreicht.

Durch diese Blockierung und die Verbindung des ersten Arbeitskanals 9 mit der Vene 25 tritt in der Arteria carotis interna 2 eine Flussumkehr auf, die durch Pfeile 13', 35 und 36 in Fig. 3 angedeutet ist. Trotz dieser Flussumkehr ist es möglich, über den zweiten Arbeitskanal des Ballonkatheters 16 in antegrader Richtung gemäß einem Pfeil 37 beispielsweise Kontrastmittel, Medikamente oder NaCl in die Arteria carotis interna 2 einzubringen. Der normale Fluss in der Arteria carotis externa 3 gemäß dem Pfeil 14 wird durch die Blockierung der Arteria carotis interna 2 nicht beeinflusst.

Nachdem das erfindungsgemäße Blockiersystem vollständig gemäß Fig. 3 positioniert ist, kann durch den ersten Arbeitskanal ein Instrument zum Verringern bzw. Entfernen der Stenose 12 eingeführt werden. Dieses kann beispielsweise als weiterer Katheter 38 mit einem Dilatationsballon 39 an seinem distalen Ende ausgebildet sein, wie es in Fig. 4 angedeutet ist. Über den Dilatationsballon 39 kann die Stenose 12 in üblicher Weise aufgeweitet werden. Es ist auch möglich, dass in bekannter Weise ein Stent in den Bereich der Stenose 12 eingebracht und platziert sowie über den Dilatationsballon 39 aufgeweitet wird. Bei Einbringen eines selbst expandierten Stents kann die Aufweitung über den Dilatationsballon 39 auch entfallen.

Trotz des eingebrachten Katheters 38 bleibt der Rückstrom durch den ersten Arbeitskanal 9 solange erhalten, solange der Strom nicht durch den vollständig aufgeweiteten Dilatationsballon 39 unterbrochen ist, wie dies in Fig. 4 durch Pfeile 40 angedeutet ist. Sollten sich bei der Aufweitung der Stenose 12 Ablagerungen 41 ablösen, so werden diese aufgrund des Rückstroms in Pfeilrichtung 40 in den ersten Arbeitskanal 9 angesaugt und führen somit nicht zu einer Embolie. Auch eventuell distal des Dilatationsballons 39 abgelöste Ablagerungen 42 werden nach Zusammenfalten des Dilatationsballons 39 und den sich daraufhin wieder einstellenden Rückstrom über den ersten Arbeitskanal 9 des Arbeitskatheters 7 abgesaugt. Dies gilt auch, wenn über den zweiten Arbeitskanal 19 in antegrader Richtung beispielsweise Kontrastmittel gemäß einem Pfeil 43 eingespritzt wird, da zum einen über den größeren Durchmesser des ersten Arbeitskanals 9 und zum anderen über die Steuerung der Strömung in antegrader Richtung durch den zweiten Arbeitskanal 19 die Strömung insgesamt so eingestellt werden kann, dass eine Hauptströmung in retrograder Richtung besteht.

Während bei dem Ausführungsbeispiel gemäß Fig. 5 der Abdichtungsballon 22 als einteiliger Ballon so ausgebildet ist, dass er den Arbeitskatheter 7 zur Abdichtung der Arteria carotis interna 2 umschließt, ist in Fig. 6 eine Ausführungsform dargestellt, bei der der Abdichtungsballon 22 aus vier Teilballons 44 bis 47 besteht. Bei der in Fig. 6 dargestellten Ausführungsform ist somit keine Ausrichtung des Ballonkatheters 16 zu dem Arbeitskatheter 7 erforderlich, um zuverlässig ein Umgreifen des Arbeitskatheters 7 und damit ein Abdichten der Arteria carotis interna 2 zu erreichen.

Während in den in den Figuren dargestellten Ausführungsbeispielen die erfindungsgemäße Vorrichtung jeweils zur Behandlung einer Stenose in der Arteria carotis interna beschrieben wurde, ist die Erfindung grundsätzlich auch bei Stenosen in sonstigen Blutgefäßen verwendbar. Die Ausführungsbeispiele sollen daher keine einschränkende Wirkung für die Verwendung speziell in der Arteria carotis interna darstellen.

### Bezugszeichenliste

- 1: Arteria carotis communis
- 2: Arteria carotis interna
- 3: Arteria carotis extrema
- 4: Zweig der Arteria femoralis
- 5: Zweig der Arteria femoralis
- 6: Zugang
- 7: Arbeitskatheter
- 8: Lumen
- 9: erster Arbeitskanal
- 10: Führungsdraht
- 11: distales Ende des Arbeitskatheters
- 12: Stenose
- 13, 13': Pfeil
- 14: Pfeil
- 15: Zugang
- 16: Ballonkatheter
- 17: Führungsdraht
- 18: Lumen
- 19: zweiter Arbeitskanal
- 20: Lumen
- 21: distales Ende des Ballonkatheters
- 22: Abdichtungsballon
- 23: proximales Ende des Arbeitskanals
- 24: Shunt
- 25: Vene
- 26: Druckerzeugungseinheit
- 27: Druckausgleichsbehälter
- 28: Ventil
- 29: Anzeige
- 30: Y-Verbindung
- 31: Ast der Y-Verbindung
- 32: Ast der Y-Verbindung
- 33: Spritze
- 34: Schleuse
- 35: Pfeil
- 36: Pfeil
- 37: Pfeil
- 38: Katheter
- 39: Dilatationsballon
- 40: Pfeil
- 41: Ablagerung
- 42: Ablagerung
- 43: Pfeil
- 44: Teilballon
- 45: Teilballon
- 46: Teilballon
- 47: Teilballon

## Patentansprüche

1. Vorrichtung zum Verringern oder Entfernen einer in einem Blutgefäß (2) vorhandenen Stenose (12) mit einem Arbeitskatheter (7) mit einem Lumen (8), das einen sich vorn distalen Ende (11) bis zum proximalen Ende (23) des Arbeitskatheters (7) erstreckenden ersten Arbeitskanal (9) zum Einführen eines Instruments (38, 39) zum Verringern oder Entfernen der Stenose (12) bildet,
wobei ein von dem Arbeitskatheter (7) getrennt ausgebildeter Ballonkatheter (16) vorgesehen ist, der im Bereich seines distalen Endes (21) einen Abdichtungsballon (22) umfaßt,
wobei der Ballonkatheter (16) zwei voneinander getrennt ausgebildete Lumen (18, 20) umfasst, von denen eines (20) zur Dilatation des Abdichtungsballons (22) mit diesem verbunden ist, während das andere (18) einen sich vom distalen Ende (21) bis zum proximalen Ende des Ballonkatheters (16) erstreckenden zweiten Arbeitskanal (19) bildet,
der Abdichtungsballon (22) und wobei zum seitlichen Umgreifen des distalen Endbereichs (23) des Arbeitskatheters (7) ausgebildet ist, so dass bei nebeneinander in das Blutgefäß (2) eingebrachtem Arbeitskatheter (7) und Ballonkatheter (16) durch den dilatierten Abdichtungsballon (22) eine Abdichtung zwischen den Außenseiten des Arbeitskatheters (7) und des Ballonkatheters (16) sowie der Innenwand des Blutgefäßes (2) gebildet wird, **dadurch gekennzeichnet, dass** der Abdichtungsballon (22) zum seitlichen Umgreifen des distalen Endbereichs des Arbeitskatheters mit einem unsymmetrischen Querschnitt ausgebildet ist.

2. Vorrichtung nach Anspruch 1,
**dadurch gekennzeichnet,**
das der Abdichtungsballon (22) eine Vielzahl von Einzelballons (44, 45,46,47) umfasst.

3. Vorrichtung nach Anspruch 1 oder 2,
**dadurch gekennzeichnet,**
**dass** eine venöse Schleuse (24) vorgesehen ist, über die der erste Arbeitskanal (9) mit einer Vene (25), verbindbar ist.

4. Vorrichtung nach einem der vorhergehenden Ansprüche,
**dadurch gekennzeichnet,**
**dass** der erste und/oder der zweite Arbeitskanal (9, 19) zum Aufnehmen eines Führungsdrahtes (10, 17) ausgebildete sind.

5. Vorrichtung nach einem der vorhergehenden Ansprüche,
**dadurch gekennzeichnet,**
**dass** der zweite Arbeitskanal (19) zum Einspritzen von Flüssigkeit, insbesondere von Kontrastmittel, von Medikamenten oder von NaCl ausgebildet ist.

6. Vorrichtung nach einem der vorhergehenden Ansprüche,
**dadurch gekennzeichnet,**
**dass** eine Druckerzeugungseinheit (26) zum Erzeugen eines konstanten Drucks vorgesehen ist, die über das Lumen (20) mit dem Abdichtungsballon (22) verbunden ist, und durch die nach Dilatation des Abdichtungsballons (22) der Druck innerhalb des Abdichtungsballons (22) im Wesentlichen konstant gehalten wird.

7. Vorrichtung nach Anspruch 6,
**dadurch gekennzeichnet,**
**dass** die Druckerzeugungseinheit (26) einen Druckausgleichsbehältier (27) umfasst.

8. Vorrichtung nach Anspruch 7,
**dadurch gekennzeichnet,**
**dass** der Druckausgleichsbehälter (27) ein wesentlich höheres Volumen besitzt als der Abdichtungsballon (22).

9. Vorrichtung nach einem der vorhergehenden Ansprüche,
**dadurch gekennzeichnet,**
**dass** der erste Arbeitskanal (9) einen größeren Durchmesser besitzt als der zweite Arbeitskanal (19).

10. Vorrichtung nach einem der vorhergehenden Ansprüche,
**dadurch gekennzeichnet,**
**dass** der Arbeitskatheter (7) einen Durchmesser von ca. 5-9 French, insbesondere von ca. 6-8 French, bevorzugt von ca. 7 French besitzt.

11. Vorrichtung nach einem der vorhergehenden Ansprüche,
**dadurch gekennzeichnet ,**
**dass** der Ballonkatheter (16) einen Durchmesser von ca. 7-14 mm, insbesondere von ca. 12 mm besitzt.

12. Vorrichtung nach einem der vorhergehenden Ansprüche,
**dadurch gekennzeichnet**,
das der Abdichtungsballon (22) eine axiale Länge von ca. 0,5-3cm, insbesondere von ca. 1-1,5cm besitzt.

13. Vorrichtung nach einem der vorhergehenden Ansprüche,
**dadurch gekennzeichnet**,
das der Abdichtungsballon (22) aus elastischem Material besteht.

14. Vorrichtung nach einem der Ansprüche 1 bis 13,
**dadurch gekennzeichnet,**
**dass** der Abdichtungsballön (22) aus unelastischem Material besteht.

15. Vorrichtung nach einem der vorhergehenden Ansprüche,
**dadurch gekennzeichnet,**
**dass** der maximale Durchmesser des Abdichtungsballons (22) größer ist als der Durchmesser des Blutgefäßes (2).

## Claims

1. A device for the reduction or the removal of a stenosis (12) present in a blood vessel (2) comprising a working catheter (7) with a lumen (8) which forms a first working channel (9) extending from the distal end (11) up to the proximal end (23) of the working catheter (7) for the introduction of an instrument (38, 39) for the reduction or the removal of the stenosis (12),
wherein a balloon catheter (16) is provided which is made separate from the working catheter (7) and which includes a sealing balloon (22) in the region of its distal end (21);
wherein the balloon catheter (16) includes two lumens (18, 20) which are made separate from one another and of which one (20) is connected to the sealing balloon (22) for the dilatation thereof, whereas the other (18) forms a second working channel (19) extending from the distal end (21) up to the proximal end of the balloon catheter (16),
and wherein the sealing balloon (22) is made to engage laterally around the distal end region (23) of the working catheter (7) so that, when the working catheter (7) and the balloon catheter 16) are introduced next to one another into the blood vessel (2), a seal is formed by the dilatated sealing balloon (22) between the outer sides of the working catheter (7) and of the balloon catheter (16) as well as the inner wall of the blood vessel (2), **characterized in that** the sealing balloon (22) is made with an asymmetrical cross-section to engage laterally around the distal end of the working catheter.

2. A device in accordance with claim 1, **characterized in that** the sealing balloon (22) includes a plurality of individual balloons (44, 45, 46, 47).

3. A device in accordance with claim 1 or 2, **characterized in that** a venous sluice (24) is provided via which the first working channel (9) can be connected to a vein (25).

4. A device in accordance with any one of the preceding claims, **characterized in that** the first and/or the second working channel (9, 19) is/are made for the reception of a guide wire (10, 17).

5. A device in accordance with any one of the preceding claims, **characterized in that** the second working channel (19) is made for the injection of liquid, in particular of contrast medium, of medication or of NaCl.

6. A device in accordance with any one of the preceding claims, **characterized in that** a pressure generating unit (26) for the generation of a constant pressure is provided which is connected via the lumen (20) to the sealing balloon (22) and via which the pressure within the sealing balloon (22) is kept substantially constant after dilatation of the sealing balloon (22).

7. A device in accordance with claim 6, **characterized in that** the pressure generating unit (26) includes a pressure compensation tank (27).

8. A device in accordance with claim 7, **characterized in that** the pressure compensation tank (27) has a substantially higher volume than the sealing balloon (22).

9. A device in accordance with any one of the preceding claims, **characterized in that** the first working channel (9) has a larger diameter than the second working channel (19).

10. A device in accordance with any one of the preceding claims, **characterized in that** the working catheter (7) has a diameter of approximately 5-9 French, in particular of approximately 6-8 French, preferably of approximately 7 French.

11. A device in accordance with any one of the preceding claims, **characterized in that** the balloon catheter (16) has a diameter of approximately 7-14 mm, in particular of approximately 12 mm.

12. A device in accordance with any one of the preceding claims, **characterized in that** the sealing balloon (22) has a axial length of approximately 0.5-3 cm, in particular of approximately 1-1.5 cm.

13. A device in accordance with any one of the preceding claims, **characterized in that** the sealing balloon (22) comprises elastic material.

14. A device in accordance with any one of the claims 1 to 13, **characterized in that** the sealing balloon (22) comprises inelastic material.

15. A device in accordance with any one of the preceding claims, **characterized in that** the maximum diameter of the sealing balloon (22) is larger than the diameter of the blood vessel (2).

## Revendications

1. Dispositif pour réduire ou pour supprimer une sténose (12) présente dans un vaisseau sanguin (2) avec un cathéter de travail (7) présentant un lumen (8), qui forme un premier canal de travail (9) s'étendant depuis l'extrémité distale (11) jusqu'à l'extrémité proximale (23) du cathéter de travail (7) pour introduire un instrument (38, 39) destiné à réduire ou à supprimer la sténose (12),
dans lequel est prévu un cathéter à ballon (16) réalisé séparément du cathéter de travail (7), qui comprend un ballon d'étanchement (22) dans la région de son extrémité distale (21),
dans lequel le cathéter à ballon (16) comprend deux lumens (18, 20) réalisés séparément l'un de l'autre, dont l'un (20) est relié au ballon d'étanchement (22) pour la dilatation de celui-ci, tandis que l'autre (18) forme un second canal de travail (19) s'étendant depuis l'extrémité distale (21) jusqu'à l'extrémité proximale du cathéter à ballon (16), et dans lequel le ballon d'étanchement (22) est réalisé pour entourer latéralement la zone d'extrémité distale (23) du cathéter de travail (7) de telle sorte que, lorsque le cathéter de travail (7) et le cathéter à ballon (16) sont introduits l'un à côté de l'autre dans le vaisseau sanguin (2), il se forme en raison du ballon d'étanchement dilaté un étanchement entre les faces extérieures du cathéter de travail (7) et du cathéter à ballon (16) ainsi que la paroi intérieure du vaisseau sanguin (2),
**caractérisé en ce que** le ballon d'étanchement (22) est réalisé pour entourer latéralement la zone d'extrémité distale du cathéter de travail avec une section transversale non symétrique.

2. Dispositif selon la revendication 1,
**caractérisé en ce que** le ballon d'étanchement (22) comprend une pluralité de ballons individuels (44, 45, 46, 47).

3. Dispositif selon la revendication 1 ou 2,
**caractérisé en ce qu'**il est prévu un sas veineux (24) via lequel le premier canal de travail (9) peut être relié avec une veine (25).

4. Dispositif selon l'une des revendications précédentes,
**caractérisé en ce que** le premier et/ou le second canal de travail (9, 19) sont réalisés pour recevoir un fil de guidage (10, 17).

5. Dispositif selon l'une des revendications précédentes,
**caractérisé en ce que** le second canal de travail (19) est réalisé pour injecter des liquides, en particulier des agents de contraste, les médicaments, ou du chlorure de sodium.

6. Dispositif selon l'une des revendications précédentes,
**caractérisé en ce qu'**il est prévu une unité de génération de pression (26) pour générer une pression constante, qui est reliée via le lumen (20) avec le ballon d'étanchement (22) et au moyen de laquelle, après dilatation du ballon d'étanchement (22), la pression à l'intérieur du ballon d'étanchement (22) est maintenue essentiellement constante.

7. Dispositif selon la revendication 6,
**caractérisé en ce que** l'unité de génération de pression (26) comprend un réservoir de compensation de pression (27).

8. Dispositif selon la revendication 7,
**caractérisé en ce que** le réservoir de compensation de pression (27) possède un volume sensiblement plus élevé que le ballon d'étanchement (22).

9. Dispositif selon l'une des revendications précédentes,
**caractérisé en ce que** le premier canal de travail (9) possède un diamètre plus élevé que le second canal de travail (19).

10. Dispositif selon l'une des revendications précédentes,
**caractérisé en ce que** le cathéter de travail (7) possède un diamètre d'environ 5 à 9 French, en particulier environ 6 à 8 French, de préférence environ 7 French.

11. Dispositif selon l'une des revendications précédentes,
**caractérisé en ce que** le cathéter à ballon (16) possède un diamètre d'environ 7 à 14 mm, en particulier d'environ 12 mm.

12. Dispositif selon l'une des revendications précédentes,
**caractérisé en ce que** le ballon d'étanchement (22) possède une longueur axiale d'environ 0,5 à 3 cm, en particulier d'environ 1 à 1,5 cm.

13. Dispositif selon l'une des revendications précédentes,
**caractérisé en ce que** le ballon d'étanchement (22) est en matériau élastique.

14. Dispositif selon l'une des revendications 1 à 13,
**caractérisé en ce que** le ballon d'étanchement (22) est en matériau inélastique.

15. Dispositif selon l'une des revendications précédentes,
**caractérisé en ce que** le diamètre maximum du ballon d'étanchement (22) est supérieur au diamètre du vaisseau sanguin (2).
